# EUROPÄISCHE PATENTSCHRIFT

(11) **EP 1 666 099 B1**
(45) Veröffentlichungstag und Bekanntmachung des Hinweises auf die Patenterteilung: **19.09.2007**
(21) Anmeldenummer: 05090296.4
(22) Anmeldetag: 26.10.2005
(51) Int. Cl.: A61Q 19/00, A61K 8/97, A61K 8/25, A61K 8/29, A61K 8/19

(54) **Kosmetisches Hautmattierungsmittel**
Cosmetic matifying agent for the skin
Agents cosmétiques matifiants pour la peau

(30) Priorität: 10.11.2004 DE 102004054926
(43) Veröffentlichungstag der Anmeldung: 07.06.2006
(73) Patentinhaber: Lancaster Group GmbH, 55116 Mainz (DE)
(72) Erfinder: Golz-Berner, Karin, 98000 Monaco (MC); Zastrow, Leonhard, 98000 Monaco (MC)
(74) Vertreter: Walter, Wolf-Jürgen

(56) Entgegenhaltungen:
- WO-A-01/35921
- WO-A-20/04017934
- US-A1- 2002 082 279
- US-A1- 2005 025 846

## Beschreibung

Die Erfindung betrifft ein kosmetisches Mittel zur Mattierung der Haut, das gleichzeitig sebumbeeinflussende Wirkung hat.

Aus der US 2002/0192169 A1 ist ein Mattierungsmittel bekannt, das eine wässrige Polytetrafluorethylendispersion mit submikrofeinen PTFE-Partikeln von etwa 0,1-30 µm enthält und für fettige Haut bevorzugt als O/W-Emulsion eingesetzt wird.

US 2002/0082279 A1 beschreibt eine Zusammensetzung zur Behandlung einer Hautkrankheit, die einen Träger, mindestens ein ätherisches Öl und einen dermatologisch aktiven Wirkstoff beinhaltet. Es ist beschrieben, dass der direkte Hauteffekt der ätherischen Öle den dermatologischen therapeutischen Effekt des Wirkstoffes verstärkt.

WO 01/35921 offenbart eine kosmetische Zusammensetzung zur. Behandlung von Hautalterungserscheinungen, die einen Anissamenextrakt in Kombination mit Retinoiden enthält.

Eine hautglättende Kosmetik auf Basis von Pflanzenextrakten beschreibt WO 2004/017934 A1. Das Kosmetikum enthält 0,01 bis 20 Gew.-% eines Puders, das u.a. Bambuspuder aus dem pulverisierten Mark von *Bambusa arundinacea* sein kann.

Die WO 2004/006872 beschreibt ein kosmetisches Mattierungsmittel mit einem wasserlöslichen oder dispergierbaren Polymeren mit LCST-Einheiten oder als Pfropfpolymeres, wobei keine anderen Füllstoffe oder Pigmente enthalten sind.

Der Erfindung liegt die Aufgabe zugrunde, ein kosmetisches Mattierungsmittel bereitzustellen, das auf Basis pflanzlicher Wirkstoffe sowohl eine mattierende als auch eine sebumbeeinflussende Wirkung hat.

Erfindungsgemäß bereitgestellt wird ein kosmetisches Hautmattierungsmittel das einen Komplex umfasst, bestehend aus 0,1-2 Gew-% eines Stengelexsudats von Bambusa arundinacea, 0,1-10 Gew-% eines Anissamenextraktes und 0,1-10 Gew-% eines Blätterextraktes von Eucalyptus, wobei der Komplex einen Anteil von 0,5 bis 12 Gew-% des Mittels hat, und der Rest bis 100 Gew-%, weitere Wirkstoffe, Trägerstoffe, Hilfsstoffe oder Gemische davon sind, wobei alle Angaben auf das Gesamtgewicht des Mittels bezogen sind.

Besonders bevorzugt ist ein Gehalt von Bambusa arundinacea im Bereich von 0,2 bis 1 Gew-%.

Weiterhin bevorzugt wird, dass der Komplex einen Anteil von 0,8 bis 6 Gew-% des Mittels einnimmt.

Das Stengelexsudat von Bambusa arundinacea, das aus der Rinde von Bambusstämmen gewonnen und auch als "Bambustränen" bezeichnet wird, wird nach dem Trocknen und Mahlen zu einem Pulver verarbeitet und kann durch Mikronisieren in eine Talkumpuder überführt werden (Hersteller Greentech Saint-Beauzire Cedex, Frankreich).

Der Anissamenextrakt von Pimpinella anisum wird mit mehrwertigen Alkoholen gewonnen, vorzugsweise mit Propylenglycol. Er zeigt u.a. antibakterielle und stimulierende Eigenschaften.

Aus den Blättern von Eucalyptus globulus (Blaugummibaum) wird ebenfalls mittels mehrwertiger Alkohole ein Extrakt gewonnen, der üblicherweise in Salben für Wunden und Abszesse, Distorsion und Muskelschmerzen aufgenommen wird.

Zusammen zeigen die drei Produkte in den angegebenen Verhältnissen eine überzeugende Mattierungswirkung zusammen mit der Fähigkeit, ohne den Zusatz von mineralischem SiO₂ oder anderem Talkumpuder Ölverbindungen in großem Umfang zu absorbieren und zwar mehr als 100 % des Eigengewichts. Damit ist dieser Komplex auch eine Alternative zu mineralischem Puder und wirkt sich darüber hinaus auch auf die Verringerung der Sebumproduktion aus, wodurch ein glänzendes Aussehen von Haut generell begrenzt werden kann.

Das erfindungsgemäße Präparat kann weiterhin kosmetische Hilfs- und Trägerstoffe enthalten, wie sie üblicherweise in solchen Zubereitungen verwendet werden, z.B. Wasser, Konservierungsmittel, Farbstoffe, Pigmente mit färbender Wirkung, Verdickungsmittel, Duftstoffe, Alkohole, Polyole, Ester, Elektrolyte, Gelbildner, polare und unpolare Öle, Polymere, Copolymere, Emulgatoren, Wachse, Stabilisatoren.

Zu den kosmetischen Wirkstoffen gehören z. B. anorganische und organische Lichtschutzmittel, Radikalfänger, Feuchthaltemittel, Vitamine, Enzyme, pflanzliche Wirkstoffe, Polymere, Antioxidationsmittel, entzündungswidrige natürliche Wirkstoffe und Erweichungsmittel.

Ein bevorzugter weiterer Wirkstoff ist beispielsweise ein mit sphärischem SiO₂ oder TiO₂ modifiziertem Kaolin gemäß WO96/17588 Beispiel 1 oder ein wässriges Wirkstoffgemisch aus einem Rindenextrakt von Quebracho blanco, einem Seidenraupenextrakt, einem Hydro-Gel und Phospholipiden (gemäß WO99/66881 Wirkstoffkomplex aus Beispiel 1), oder ein oder mehrere Antioxidationsmittel, Flavone, Flavonoide oder Gemische von Wirkstoffen dieser Gruppe.

Zu Antioxidationsmitteln und Radikalfängern gehören Vitamine wie Vitamin C und Derivate davon, beispielsweise Ascorbylacetate, -phosphate und -palmitate; Vitamin A und Derivate davon; Folsäure und deren Derivate, Vitamin E und deren Derivate, wie Tocopherylacetat; Flavone oder Flavonoide; Aminosäuren, wie Histidin, Glycin, Tyrosin, Tryptophan und Derivate davon; Carotinoide und Carotine, wie z.B α-Carotin, β-Carotin; Harnsäure und Derivate davon; α-Hydroxysäuren wie Citronensäure, Milchsäure, Apfelsäure; Stilbene und deren Derivate.

Es ist weiterhin vorteilhaft, den erfindungsgemäßen Zusammensetzungen entsprechende wasser- und/oder öllösliche UVA- oder UVB-Filter oder beide zuzusetzen. Zu vorteilhaften öllöslichen UVB-Filtern gehören 4-Aminobenzoesäure-Derivate wie der 4-(Dimethylamino)-benzoesäure-(2-ethylhexyl)ester; Ester der Zimtsäure wie der 4-Methoxyzimtsäure(2-ethylhexyl)ester, Benzophenon-Derivate wie 2-Hydroxy-4-methoxybenzophenon; 3-Benzylidencampher-Derivate wie 3-Benzylidencampher.

Wasserlösliche UVB-Filter sind z.B. Sulfonsäurederivate von Benzophenon oder von 3-Benzylidencampher oder Salze wie das Na- oder K-Salz der 2-Phenylbenzimidazol-5-sulfonsäure.

Zu UVA-Filtern gehören Dibenzoylmethan-Derivate wie 1-Phenyl-4-(4'-isopropylphenyl)propan-1,3-dion, Butyl Methoxybenzoylmethane oder Menthyl Anthranilate.

Besonders bevorzugt sind Benzophenone-3, Butyl Methoxydibenzoylmethane, Octyl Methoxycinnamate, Octyl Salicylate, 4-Methylbenzylidene Camphor, Homosalate, Octocrylene, Ethylhexyl Methoxycinnamate, Isoamyl-p-Methoxycinnamate, Octyl Dimethyl PABA, Ethylhexyltriazone, Diethylhexyl Butamido Triazone, Ethylhexyl Salicylate, Methylene Bis-Benzotriazolyl Tetramethylbutylphenol, Disodium Phenyl Dibenzimidazole Tetrasulfonate, Bis-Ethylhexyloxyphenol Methoxyphenyl Triazine. Weiterhin einsetzbar als Sonnenschutzfilter sind anorganische Pigmente auf Basis von Metalloxiden, wie TiO₂, SiO₂, ZnO, Fe₂O₃, ZrO₂, MnO, Al₂O₃, die auch im Gemisch verwendet werden können.

Kosmetische Zusammensetzungen mit der erfindungsgemäßen Wirkstoffzubereitung können als O/W- oder W/O-Emulsionen vorliegen. Geeignete Emulgatoren für O/W-Emulsionen sind beispielsweise Anlagerungsprodukte von 2-30 Mol Ethylenoxid an lineare C₈-C₂₂-Fettalkohole, an C₁₂-C₂₂-Fettsäuren und an C₈-C₁₅-Alkylphenole; C₁₂-C₂₂-Fettsäuremono- und -diester von Anlagerungsprodukten von 1-30 Mol Ethylenoxid an Glycerin.

Geeignete Emulgatoren für W/O-Emulsionen sind beispielsweise Anlagerungsprodukte von 2-15 Mol Ethylenoxid an Ricinusöl; Ester von C₁₂-C₂₂-Fettsäuren und Glycerin, Polyglycerin, Pentaerythrit, Zuckeralkohole (z.B. Sorbit), Polyglucoside (z.B. Cellulose); Polyalkylenglycole; Wollwachsalkohole; Copolymere von Polysiloxan-Polyalkylpolyether.

Die für die Erfindung eingesetzten Öle können übliche kosmetische Öle sein, wie ein Mineralöl; hydriertes Polyisobuten; synthetisches oder aus Naturprodukten hergestelltes Squalan; kosmetische Ester oder Ether, die verzweigt oder unverzweigt, gesättigt oder ungesättigt sein können; pflanzliche Öle; oder Gemische zweier oder mehrerer davon.

Besonders geeignete Öle sind beispielsweise Siliconöle, Mineralöle, Hydrogenated Polyisobuten, Polyisopren, Squalane, Tridecyltrimellitat, Trimethylpropantriisostearat, Isodecylcitrat, Neopentylglycoldiheptanoat, PPG-15-stearylether sowie pflanzliche Öle, wie Calendulaöl, Jojobaöl, Avocadoöl, Macadamianußöl, Rizinusöl, Kakaoobutter, Kokosnußoil, Maisöl, Baumwollsamenöl, Olivenöl, Palmkernöl, Rapssamenöl, Safloröl, Sesamsamenöl, Sojabohnenöl, Sonnenblumensamenöl, Weizenkeimöl, Traubenkernöl, Kukuinußöl, Distelöl und Gemische davon.

Je nachdem welche Öle ausgewählt werden, werden die kosmetischen Eigenschaften der festen Zusammensetzung beeinflußt, wie Transparenzgrad, Weichheit, Härte, Spreitungswirkung.

Als Erweichungsmittel können normalerweise eine Vielzahl von Verbindungen eingesetzt werden, wie Stearylalkohol, Glycerylmonoricinoleat, Glycerylmonostearat, Propan-1,2-diol, Butan-1,3-diol, Cetylalkohol, Isopropylisostearat, Stearinsäure, Isobutylpalmitat, Isopropylmyristat, Isopropylpalmitat, Oleylalkohol, Isopropyllaurat, Decyloleat, Octadecan-2-ol, Isocetylalkohol, Cetylpalmitat, Siliconöle wie Dimethylpolysiloxan, Polyethylenglycol, Lanolin, Kakaobutter, pflanzliche Öle wie Maisöl, Baumwollsamenöl, Olivenöl, mineralische Öle, Butylmyristat, Palmitinsäure usw.

Geeignete Feuchthaltemittel sind beispielsweise Glycerin, Butylenglycol, Propylenglycol und Gemische davon.

Als Farbpigmente, Pigmentgemische oder Pulver mit pigmentartiger Wirkung, worunter auch solche mit Perlglanz-Effekt zu verstehen sind, können zum Beispiel eingesetzt werden: Eisenoxide, natürliche Aluminiumsilicate wie Ocker, Titan(di)oxid, Glimmer, Kaolin, manganhaltige Tone wie Umbra und roter Bolus, Calciumcarbonat, Talkum, Glimmer-Titanoxid, Glimmer-Titanoxid-Eisenoxid, Glimmer-Titanoxid-organischer Farbstoff und Gemische davon.

Das erfindungsgemäße Kosmetikum hat sowohl eine ölabsorbierende Wirkung ohne Zusatz von Mineralpulvern oder organischen Polymeren als auch eine auf die Sebumbildung gerichtete Wirkung, indem es die Lipidbalance im Sebum hält. Diese Lipidbalance liegt bei etwa 58 % Triglyceride, 26 % Wachs, 12 % Squalen und 4 % Sterole, wobei diese Werte stark von der Größe der Talgdrüsen abhängen. Bei fettiger Haut wird diese Balance in Richtung auf höhere Anteile Wachs und Squalen verschoben. Überraschenderweise wirkt der erfindungsgemäße Komplex von pflanzlichen Produkten dem entgegen und zeigt insgesamt eine ausgezeichnete mattierende Wirkung. Dies wurde auch durch Tests bestätigt.

Die Verwendung der erfindungsgemäßen kosmetischen Zusammensetzungen kann z.B. erfolgen in Form von Sonnencremes, Sonnengelen, After-sun-Produkten, Tagescremes, Nachtcremes, Körperlotionen, Reinigungsmilch, Körperpuder, Augenkosmetik, und in Produkten der dekorativen Kosmetik wie Deo-Stiften, Parfüm-Stiften, Lippenstiften, Gelen, Lidschatten, Kompaktprodukten wie Kompaktpuder oder Kompaktwachs, Rouge, Grundierung, Make-up usw. Die Herstellung derartiger Produkte erfolgt auf eine Weise, wie sie dem Fachmann auf diesem Gebiet bekannt ist.

Die Herstellung des Mattierungskomplexes erfolgt durch einfaches Vermischen der Einzelbestandteile in einem flüssigen Träger wie zum Beispiel Wasser, Glycerin, Propylenglycol oder Gemischen davon. Die Erfindung soll nachstehend durch Beispiele näher erläutert werden. Alle Angaben erfolgen in Gewichtsprozent, sofern nichts anderes angegeben ist.

**Beispiel 1 Sommercreme**

| **Phase A** | |
|---|---|
| Wasser | q.s. ad 100 |
| Carbomere | 0,5 |
| Glycerin | 2,5 |

| **Phase B** | |
|---|---|
| Silicone | 5,0 |
| Jojobaöl | 10,0 |

| **Phase C** | |
|---|---|
| Triethanolamin | 0,5 |

| **Phase D** | |
|---|---|
| RPF-Komplex¹ | 1,0 |
| Kaolin^{2*} | 1,0 |
| Mattierungskomplex³ | 5,0 |
| Konservierungsmittel | 0,5 |
| Parfümöl | 0,5 |

| | |
|---|---|
| ¹ gemäß WO99/66881 Wirkstoffkomplex aus Beispiel 1 ² mit sphärischen SiO₂-Teilchen gemäß WO96/17588 Beispiel 1 ³ Bambusa arundinacea 0,5%, Anis 5,0%, Eucalyptus globulus 4,5% | |

Die separat hergestellten Phasen A und B werden auf 60 °C erwärmt und zusammengegeben. Das ausdauernd gerührte Gemisch wird auf 55 °C abgekühlt, die Phase C wird unter Rühren hinzugegeben, und nach Abkühlung auf 35 °C wird Phase D hinzugegeben und weiter gerührt.

**Beispiel 2 Tagescreme**

| **Phase A** | |
|---|---|
| Wasser | q.s. ad 100 |
| Carbomere | 0,5 |
| Propylenglcol | 5,0 |

| **Phase B** | |
|---|---|
| Silicone | 3,0 |
| Jojobaöl | 2,0 |
| Babassuöl | 2,0 |

| **Phase C** | |
|---|---|
| Triethanolamin | 0,5 |

| **Phase D** | |
|---|---|
| RPF-Komplex¹ | 1,5 |
| Kaolin² | 0,5 |
| Talkum | 0,5 |
| Mattierungskomplex³ | 5,0 |
| Konservierungsmittel | 0,5 |
| Parfümöl | 0,8 |

| | |
|---|---|
| ¹ gemäß WO99/66881 Wirkstoffkomplex aus Beispiel 1 ² mit sphärischen SiO₂-Teilchen gemäß WO96/17588 Beispiel 1 ³ Bambusa arundinacea 0,3%, Anis 2,1%, Eucalyptus globulus 2,6% | |

Die Verfahrensweise entspricht der von Beispiel 1.

**Beispiel 3 Körperlotion**

| **Phase A** | |
|---|---|
| Wasser | q.s. ad 100 |
| Carbomere | 0,2 |
| Glycerin | 5,0 |

| **Phase B** | |
|---|---|
| Silicone | 1,0 |
| Jojobaöl | 5,0 |

| **Phase C** | |
|---|---|
| Triethanolamin | 0,2 |

| **Phase D** | |
|---|---|
| Mattierungskomplex³ | 2,0 |
| Konservierungsmittel | 0,5 |
| Parfümöl | 0,5 |
| Beispiel 1 | |

| | |
|---|---|
| ³ Bambusa arundinacea 0,2%, Anis 0,9%, Eucalyptus globulus 0,9% | |

Die Verfahrensweise entspricht der von Beispiel 1.

### Beispiel 4 Vergleichsversuche

### a) Reflektometertest

Die Mattierungswirkung wird durch Aufsprühen eines entsprechenden Mittels auf einen stumpfen Träger, z.B. einen Kunststoff oder einen Gummi gemessen. Das mit einer Konzentration von 2-3 g/cm² aufgesprühte und getrocknete Material wird mittels Gonioreflektometer gemessen und dabei wird das Verhältnis von Spiegelreflexion und Diffusreflexion ermittelt. Die mattierenden Zusammensetzungen zeigen dabei kleinere Werte als nicht-mattierende Zusammensetzungen.

Die Zusammensetzung gemäß Beispiel 1 zeigt einen Wert für dieses Verhältnis von 1,93, Beispiel 2 von 1,85 und Beispiel 3 von 1,88.

Der Vergleich mit der Basiszusammensetzung des Kosmetikums, jeweils ohne den Komplex, ergibt einen durchschnittlichen Wert von 2,8.

### b) Verbrauchertest

Die Mattierungszusammensetzung gemäß Beispiel 1 wurde darüber hinaus einem Verbrauchertest unterzogen. Eine Gruppe von Probanden mit fettiger Haut trug einmal täglich für 7 Tage auf die Stirnfläche eine Mattierungscreme gemäß Beispiel 1 auf. Nach 8 Tagen und vorheriger üblicher Gesichtswäsche spätestens 4 Stunden vor einer visuellen Begutachtung wurde der verbliebene Mattierungseffekt unter Beleuchtung mit einer normalen Glühlampe (75 W) im Winkel von 45° visuell eingeschätzt und mit den nichtbehandelten Wangenpartien verglichen.
1: kein Mattierungseffekt
2: geringer Mattierungseffekt
3: deutlicher Mattierungseffekt
4: starker Mattierungseffekt.

Der gebildete Durchschnittswert der Probandengruppe lag bei 2,75, also einem noch als deutlich zu bezeichnenden Mattierungseffekt auch 24 Stunden nach Absetzen der Behandlung. Dies lässt auf einen wirksamen Einfluss auf die Lipidbalance schließen.

## Patentansprüche

1. Kosmetisches Hautmattierungsmittel, **dadurch gekennzeichnet, dass** es einen Komplex umfasst, bestehend aus 0,1-2 Gew-% eines Stengelexsudats von Bambusa arundinacea, 0,1-10 Gew-% eines Anissamenextraktes und 0,1-10 Gew-% eines Blätterextraktes von Eucalyptus, wobei der Komplex einen Anteil von 0,5 bis 12 Gew-% des Mittels hat, und der Rest bis 100 Gew-% weitere Wirkstoffe, Trägerstoffe, Hilfsstoffe oder Gemische davon sind, wobei alle Angaben auf das Gesamtgewicht des Mittels bezogen sind.

2. Mittel nach Anspruch 1, **dadurch gekennzeichnet, dass** das der Gehalt von Bambusa arundinacea im Bereich von 0,2 bis 1 Gew-% liegt.

3. Mittel nach Anspruch 1, **dadurch gekennzeichnet, dass** der Komplex einen Anteil von 0,8 bis 6 Gew-% des Mittels hat.

4. Mittel nach Anspruch 1, **dadurch gekennzeichnet, dass** ein weiterer Wirkstoff aus der Gruppe ausgewählt ist, bestehend aus mit sphärischem SiO₂ oder TiO₂ modifiziertem Kaolin; einem wässrigen Wirkstoffgemisch aus einem Rindenextrakt von Quebracho blanco, einem Seidenraupenextrakt, einem Hydro-Gel und Phospholipiden; Antioxidationsmitteln; Flavonen; Flavonoiden; und Gemischen davon.

## Claims

1. A cosmetic skin matting agent, **characterized in that** it comprises a complex consisting of 0.1 to 2 wt.-% of a stem exudate from Bambusa arundinacea, 0.1 to 10 wt.-% of an aniseed extract and 0.1 to 10 wt.-% of an extract of eucalyptus leaves, said complex representing a share of 0.5 to 12 wt.-% in said agent, and the balance to make 100 wt.-% being further active substances, excipients, auxiliaries or mixtures thereof, the above quantities being based on the overall weight of the agent.

2. The agent according to claim 1, **characterized in that** the content of Bambusa arundinacea ranges from 0.2 to 1 wt.-%.

3. The agent according to claim 1, **characterized in that** the complex represents a share of from 0.8 to 6 wt.-% in said agent.

4. The agent according to claim 1, **characterized in that** an additional active substance is selected from the group consisting of kaolin modified with spherical SiO₂ or TiO₂; an aqueous mixture of active substances from a bark extract of Quebracho blanco, a silkworm extract, a hydrogel and phospholipids; antioxidants; flavones; flavonoids; and mixtures thereof.

## Revendications

1. Agent matifiant de la peau, **caractérisé en ce qu'**il comprend un complexe, constitué de 0,1 à 2 % en poids d'un exsudat de tige de Bambusa arundinacea, 0,1 à 10 % en poids d'un extrait de graine d'anis et 0,1 à 10 % en poids d'un extrait de feuilles d'eucalyptus, moyennant quoi le complexe représente une proportion de 0,5 à 12 % en poids de l'agent, et le reste jusqu'à 100 % en poids est constitué de principes actifs, de substances de support, d'adjuvants complémentaires ou de mélanges d'entre eux, toutes les quantités se rapportant au poids total de l'agent.

2. Agent selon la revendication 1, **caractérisé en ce que** la teneur en Bambusa arundinacea est comprise dans la plage de 0,2 à 1 % en poids.

3. Agent selon la revendication 1, **caractérisé en ce que** le complexe représente une proportion de 0,8 à 6 % en poids de l'agent.

4. Agent selon la revendication 1, **caractérisé en ce qu'**on sélectionne un principe actif complémentaire dans le groupe comprenant le kaolin modifié avec du SiO₂ ou TiO₂ sphérique; un mélange de principes actifs aqueux constitué d'un extrait d'écorce de Quebracho blanco, d'un extrait de ver à soie, d'un hydrogel et de phospholipides; des agents antioxydants; des flavones; des flavonoïdes; et des mélanges d'entre eux.
